# EUROPEAN PATENT SPECIFICATION

(11) **EP 3 556 368 B1**
(45) Date of publication and mention of the grant of the patent: **26.05.2021**
(21) Application number: 17881795.3
(22) Date of filing: 15.12.2017
(51) Int. Cl.: A61K 31/454, A61K 47/04, A61K 47/08, A61K 47/10, A61K 47/14, A61K 47/18, A61K 47/38, A61P 17/00, A61P 31/10, A61K 9/70, A61K 47/22

(54) **ONYCHOMYCOSIS THERAPEUTIC AGENT**
THERAPEUTIKUM FÜR ONYCHOMYKOSE
AGENT THÉRAPEUTIQUE CONTRE L'ONYCHOMYCOSE

(30) Priority: 16.12.2016 JP 2016244478
(43) Date of publication of application: 23.10.2019
(73) Proprietor: Kaneka Corporation, Osaka-shi, Osaka 530-8288 (JP)
(72) Inventor: MIZUTANI Masaya, Takasago-shi Hyogo 676-8688 (JP); TANAKA Tatsuyoshi, Takasago-shi Hyogo 676-8688 (JP); OGINO Hiroyuki, Takasago-shi Hyogo 676-8688 (JP); AKAZAWA Mitsuji, Takasago-shi Hyogo 676-8688 (JP)
(74) Representative: Hoffmann Eitle
(86) International application number: PCT/JP2017/045062
(87) International publication number: WO 2018/110693

(56) References cited:
- EP-A1- 3 505 218
- WO-A1-03/105903
- JP-A- 2002 097 129
- JP-A- 2002 542 326
- JP-A- 2004 083 439
- US-A1- 2005 186 161
- US-A1- 2014 370 078
- US-A1- 2015 099 785
- KEITA SUGIURA ET AL: "The Low Keratin Affinity of Efinaconazole Contributes to Its Nail Penetration and Fungicidal Activity in Topical Onychomycosis Treatment", ANTIMICROBIAL AGENTS AND CHEMOTHERAPY, vol. 58, no. 7, 1 July 2014 (2014-07-01), pages 3837-3842, XP055689441, US ISSN: 0066-4804, DOI: 10.1128/AAC.00111-14
- Parchem.Com: "PCA ethyl cocoyl arginate supplier distributor- CAS 95370-65-3", , 24 April 2010 (2010-04-24), pages 1-7, XP055689551, Retrieved from the Internet: URL:https://www.parchem.com/chemical-suppl ier-distributor/PCA-ethyl-cocoyl-arginate- 098520.aspx [retrieved on 2020-04-27]
- ABE, TAKASHI: "Over external pharmaceuticals and cosmetics for nails recently released", FRAGRANCE JOURNAL, vol. 42, no. 11, 2014, pages 111-112, XP009515109,
- Anonymous: "N-coconut fatty acid acyl-L-arginine ethyl DL-pyrrolidonecarboxylate", Japanese Pharmaceutical Excipients Directory 2016, 18 February 2016 (2016-02-18), page 592, XP009515431, ISBN: 978-4-8408-1329-7

## Description

### Technical Field

The present invention relates to a preparation that can be used for the treatment of onychomycosis.

### Background Art

Onychomycosis is an infection of the nail mainly caused by causative pathogens such as *Trichophyton rubrum* or *Trichophyton mentagrophytes,* which are classified into dermatophytes. This disease has been known as "athlete's foot of the nails." If a person is affected with onychomycosis, in addition to changes in the appearance, such as deformation of the nail, it becomes difficult for the person to walk due to thickening of nails. Thus, physical and/or mental burden is imposed on the patient. In addition, if onychomycosis is left without being treated, it may result in spread of the infection.

As therapeutic agents for onychomycosis, internal medicines and external medicines applied to nails have been known. As an external medicine applied to onychomycosis, a liquid agent for external use has been approved and sold for the first time in 2014 in Japan, and the market has been expanding. This liquid agent for external use is a preparation comprising efinaconazole as an active ingredient. Efinaconazole has low affinity with keratin which is a component constituting the nail, and high permeability through the nail. Accordingly, it is said that efinaconazole is highly useful as an external medicine for nails (Non Patent Literature 1). However, the existing efinaconazole-containing liquid agent for external use still has problems specific to liquid agents for external use, such that, upon the application thereof, the agent liquid may be dropped even on the skin and may cause skin irritation, or such that the applied agent liquid may be exposed to non-application sites.

As a dosage form that solves the aforementioned problems of liquid agents for external use, a semi-solid preparation, the surface of which is solidified (film formation) after the application thereof (hereinafter referred to as "film-forming preparation"), has been known (Patent Literatures 1 to 6). In such a film-forming preparation, a volatile solvent contained in the preparation is evaporated after the application thereof, so that a non-volatile component such as a solidifying agent forms a solidified layer on the applied surface.

Moreover, an external-use antifungal agent for nails, comprising neticonazole or a salt thereof and a basic substance, has been known (Patent Literature 7). Example 2 of Patent Literature 7 discloses a nail lacquer comprising neticonazole hydrochloride, ethyl cellulose, diethyl sebacate, polyoxyethylene (9) lauryl ether, diisopropanolamine, dibutylhydroxytoluene, acetone and ethanol.

Compositions containing efinaconazole, butylated hydroxytoluene, a salt of ethylenediaminetetraacetic acid, and optional citric acid have been reported (Patent Literature 8). The compositions are reported to exhibit stable color profiles and are said to be useful in the treatment of fungal infections.

Detergent compositions for the treatment of greasy and acneic skins comprising a mixture of surfactants have been reported (Patent Literature 9). The composition comprises a) sodium cocoyl alaninate; b) cocoyl methyl glucamide;c) laureth-7 citrate; d) PCA ethyl cocoyl arginate. The ingredient PCA ethyl cocoyl arginate is commercially available (see Non-Patent Literature 3)

An antifungal and/or antimycotic external preparation for nail comprising neticonazole or a salt thereof and a basic substance has been reported (Patent Literature 10).

Compositions and methods for the prevention and treatment of chronic wounds such as pressure ulcers and diabetic ulcers have been reported (Patent Literature 11). The compositions exert their therapeutic effect by transdermal delivery of an agent that increases activity of HIF-1α in the wound.

Sugiura et al. have reported that the high nail permeability of efinaconazole and its potent fungicidal activity in the presence of keratin are related to its low keratin affinity, which may contribute to its efficacy in onychomycosis (Non-patent Literature 2).

### Prior Art Literatures

### Patent Literatures

Patent Literature 1: JP Patent Publication (Kohyo) No. 2008-501805 A
Patent Literature 2: JP Patent Publication (Kohyo) No. 2009-519958 A
Patent Literature 3: JP Patent Publication (Kohyo) No. 2015-521996 A
Patent Literature 4: WO2014/104149
Patent Literature 5: JP Patent Publication (Kokai) No. 2016-27036 A
Patent Literature 6: WO2003/105903
Patent Literature 7: JP Patent Publication (Kokai) No. 2004-83439 A
Patent Literature 8: US 2015/099785 A1
Patent Literature 9: EP 3 505 218 A1
Patent Literature 10: US 2005/186161 A1
Patent Literature 11: US 2014/370078 A1

### Non Patent Literatures

Non Patent Literature 1: Folia Pharmacologica Japonica 145, 250-258 (2015)
Non Patent Literature 2: Antimicrobial Agents and Chemotherapy,58(7), 3837-3842 (2014)
Non-Patent Literature 3: Commercial brochure from Parchem cited as XP055689551 during prosecution (assigned a date of 24 April 2010)

### Summary of Invention

### Object to be Solved by the Invention

By the way, the film-forming preparation is required to have properties such as "a short time required until film formation" and "sufficient preparation cohesiveness (peeling ability) after completion of the solidification, so that the film can be easily peeled (can be removed) without breaking the film." Moreover, if the surface of the film-forming preparation is sticky after completion of the solidification, it causes problems such that the preparation is easily dropped when it comes into contact with non-application sites. Besides, in the aforementioned patent literatures, the stickiness on the surface after solidification is not explained at all.

Hence, it is an object of the present invention to provide an efinaconazole-containing film-forming preparation, which has no sticky surface after solidification thereof, has sufficient cohesiveness after solidification thereof, and is easily peeled without washing.

### Means for Solving the Object

As a result of intensive studies directed toward achieving the aforementioned object, the present inventors have revealed that an efinaconazole-containing film-forming preparation, which has no sticky surface after solidification thereof, has sufficient cohesiveness after solidification thereof, and is easily peeled without washing, can be realized by adding a surfactant to an ethyl cellulose base. In general, a surfactant as a pharmaceutical additive is used as a drug absorption promoter. However, surprisingly, the present inventors have found that when a surfactant is added to an efinaconazole-containing preparation comprising ethyl cellulose as a base, stickiness is not generated on the surface of the preparation after formation of a film, and thus, a peelable preparation having good cohesiveness can be obtained, thereby completing the present invention. Specifically, the gist of the present invention is as follows.
(1) A therapeutic preparation for onychomycosis, comprising:
   a) efinaconazole or a salt thereof,
   b) 1% by weight or more and 30% by weight or less of ethyl cellulose,
   c) 20% by weight or more of a volatile solvent, wherein the volatile solvent is water or a liquid component having a boiling point lower than a boiling point of water, and
   d) 0.01% by weight or more and less than 30% by weight of a surfactant.
(2) The preparation according to (1), wherein an amount of the surfactant in the preparation is 0.1% by weight or more and 25% by weight or less.
(3) The preparation according to (1), wherein an amount of the surfactant in the preparation is 1% by weight or more and 20% by weight or less.
(4) The preparation according to any one of (1) to (3), wherein an amount of the ethyl cellulose in the preparation is 5% by weight or more and 25% by weight or less.
(5) The preparation according to any one of (1) to (3), wherein an amount of the ethyl cellulose in the preparation is 10% by weight or more and 20% by weight or less.
(6) The preparation according to any one of (1) to (5), wherein an amount of the efinaconazole or a salt thereof in the preparation is 0.1% by weight or more and 30% by weight or less.
(7) The preparation according to any one of (1) to (5), wherein an amount of the efinaconazole or a salt thereof in the preparation is 0.5% by weight or more and 25% by weight or less.
(8) The preparation according to any one of (1) to (5), wherein an amount of the efinaconazole or a salt thereof in the preparation is 1% by weight or more and 20% by weight or less.
(9) The preparation according to any one of (1) to (8), wherein the surfactant comprises a cationic surfactant.
(10) The preparation according to (9), wherein the cationic surfactant is N-coconut oil fatty acid acyl-L-arginine-ethyl DL-pyrrolidone carboxylate.
(11) The preparation according to any one of (1) to (10), wherein the surfactant comprises an anionic surfactant.
(12) The preparation according to any one of (1) to (11), wherein the surfactant comprises an amphoteric surfactant.
(13) The preparation according to any one of (1) to (12), wherein the surfactant comprises a nonionic surfactant.
(14) The preparation according to any one of (1) to (13), wherein the surfactant has a glycerin backbone or a propylene glycol backbone.
(15) The preparation according to any one of (1) to (14), wherein the surfactant has a sorbitan backbone.
(16) The preparation according to any one of (1) to (15), wherein the volatile solvent comprises at least one selected from the group consisting of ethanol, isopropanol, ethyl acetate, acetone, diethyl ether, dimethyl ether, and water.
(17) The preparation according to any one of (1) to (16), wherein the volatile solvent comprises at least one volatile solvent other than water.
(18) The preparation according to (17), wherein an amount of the at least one volatile solvent other than water in the preparation is 50% by weight or more and 90% by weight or less.
(19) The preparation according to any one of (1) to (18), further comprising a non-volatile component other than the surfactant.
(20) The preparation according to (19), wherein an amount of the non-volatile component other than the surfactant in the preparation is 7% by weight or less.
(21) The preparation according to (19) or (20), wherein the non-volatile component other than the surfactant comprises an ester.
(22) The preparation according to (19) or (20), wherein the non-volatile component other than the surfactant comprises an alcohol.
(23) The preparation according to any one of (1) to (22), which is a liquid agent for external use, and is a film forming agent that solidifies after it has been applied to an effected area.

### Advantageous Effects of Invention

According to the present invention, an efinaconazole-containing film-forming preparation, which has no sticky surface after solidification thereof, has sufficient cohesiveness after solidification thereof, and can be easily peeled without washing, can be provided.

### Embodiments for Carrying out the Invention

Hereinafter, the present invention will be described in detail. The storage form of the preparation of the present invention is not particularly limited, and the preparation is preferably stored in a tube or an ointment jar.

The preparation of the present invention comprises (a) efinaconazole or a salt thereof as a drug, and further, at least, (b) ethyl cellulose, (c) a volatile solvent, and (d) a surfactant. The preparation of the present invention is preferably a liquid agent for external use, and also a film-forming agent that solidifies after it has been applied to an affected area.

### (a) Efinaconazole or a salt thereof

Efinaconazole that can be used in the present invention may be either free-form efinaconazole or a pharmaceutically acceptable salt of efinaconazole. Efinaconazole is also referred to as KP-103 or (2R,3R)-2-(2,4-difluorophenyl)-3-(4-methylenepiperidin-1-yl)-1-(1H-1,2,4-triazol-1-yl)but an-2-ol. Herein, the pharmaceutically acceptable salt of efinaconazole is not particularly limited, and it may be either an inorganic salt or an organic salt. Examples of the inorganic salt of efinaconazole include hydrochloride, hydrobromide, nitrate, sulfate, and phosphate. Examples of the organic salt of efinaconazole include formate, acetate, trifluoroacetate, propionate, lactate, tartrate, oxalate, fumarate, maleate, citrate, malonate, methane sulfonate, and toluene sulfonate. From the viewpoint of availability, free-form efinaconazole is preferably used.

As such efinaconazole or a salt thereof, efinaconazole or a salt thereof produced according to a known method can be used, or a commercially available product satisfying the above-described properties can also be used. In addition, efinaconazole is commercially available, and it can be purchased, for example, from Sigma-Aldrich Japan (Product code: SML1244), etc.

In order to obtain sufficient medicinal efficacy, the content of efinaconazole is preferably 0.1% by weight or more, more preferably 0.5% by weight or more, further preferably 1% by weight or more, still further preferably 2% by weight or more, particularly preferably 5% by weight or more, and most preferably 7% by weight or more, with respect to the total amount of the components of the preparation. On the other hand, from the viewpoint of ensuring viscosity easing the application of a preparation, the upper limit of the content of efinaconazole is preferably 30% by weight or less, more preferably 25% by weight or less, further preferably 20% by weight or less, particularly preferably 15% by weight or less, and most preferably 10% by weight or less.

### (b) Ethyl cellulose

Ethyl cellulose that can be used in the preparation of the present invention is not particularly limited, as long as it may be ethyl cellulose, in which the hydrogen atoms (H) of hydroxyl groups (OH group) of cellulose are substituted with ethyl groups (C₂H₅). In the present description, the percentage at which the hydrogen atoms of hydroxyl groups in ethyl cellulose are substituted with ethyl groups is referred to as "ethyl group substitution percentage."

The above-described ethyl group substitution percentage is not particularly limited, and it may be 10% or more, or 20% or more, or 30% or more, or 40% or more, or 45% or more. In addition, the ethyl group substitution percentage may be 100%, or 90% or less, or 80% or less, or 70% or less, or 60% or less, or 55% or less. From the viewpoint of solubility in a volatile solvent, etc., the ethyl group substitution percentage is preferably 30% or more and 70% or less, more preferably 40% or more and 60% or less, and further preferably 45% or more and 55% or less.

The weight average molecular weight (MW) of the above-described ethyl cellulose is not particularly limited, and it may be 20000 or more, or 40000 or more, or 60000 or more. On the other hand, it may be 400000 or less, or 300000 or less, or 230000 or less. In the present invention, a preparation having viscosity that eases the application thereof can be obtained by appropriately selecting the weight average molecular weight of ethyl cellulose and the content of ethyl cellulose with respect to the total amount of the components of the preparation.

Ethyl cellulose used in the present invention may be one type, or two or more types of ethyl cellulose, in which the ethyl group substitution percentage or the weight average molecular weight is different from one another, may be used in combination.

In a certain example of the present invention, there may be a case where ethyl cellulose is not dissolved in a volatile solvent if the ethyl group substitution percentage in ethyl cellulose is low. This may cause gelatinization or the like. On the other hand, in another example of the present invention, there may be a case where ethyl cellulose is not dissolved in a volatile solvent if the ethyl group substitution percentage is high.

In one example of the present invention, there may be a case where a preparation does not have sufficient viscosity and thus, it becomes difficult to apply the preparation, if the weight average molecular weight of ethyl cellulose is low. In another example of the present invention, there may be a case where a preparation becomes hard and thus, it becomes difficult to apply the preparation, if the weight average molecular weight of ethyl cellulose is high.

Ethyl cellulose produced by a known method can be used, or a commercially available product satisfying the above-described properties can also be used. Moreover, ethyl cellulose is commercially available, and examples of the commercially available ethyl cellulose include: "ETHOCEL STD4," "ETHOCEL STD7," "ETHOCEL STD10," "ETHOCEL STD20," "ETHOCEL STD45," "ETHOCEL STD100," "ETHOCEL STD200," "ETHOCEL STD300," "ETHOCEL MED50," and "ETHOCEL MED70," which are manufactured by The Dow Chemical; and "T10 Pharm," "N7 Pharm," "N10 Pharm," "N14 Pharm," "N22 Pharm," "N50 Pharm," and "N100 Pharm," which are manufactured by Ashland.

The content of ethyl cellulose with respect to the total amount of the components of the preparation is not particularly limited, as long as it is within the range that does not impair the applicability of the preparation as a coating agent. In the present invention, the lower limit of the content of ethyl cellulose is 1% by weight or more, more preferably 5% by weight or more, and particularly preferably 10% by weight or more, with respect to the total amount of the components of the preparation. If the content of ethyl cellulose is too high, the preparation becomes hard and thus it becomes difficult to apply the preparation. The upper limit of the content ethyl cellulose is 30% by weight or less, more preferably 25% by weight or less, and most preferably 20% by weight or less, with respect to the total amount of the components of the preparation.

### (c) Volatile solvent

The volatile solvent that can be used in the present invention is water or a liquid component having a boiling point lower than a boiling point of water. Examples of the volatile solvent include water, ethanol, isopropanol, ethyl acetate, and acetone. The volatile solvent is particularly preferably ethanol.

If the content of the volatile solvent used in the present invention is too low, the preparation becomes hard. On the other hand, if the content of the volatile solvent is too high, it is dropped upon the application thereof, and thus, it becomes difficult to apply the preparation. The content of the volatile solvent is not particularly limited, as long as it is within the range that does not impair the applicability of the preparation. The lower limit of the content of the volatile solvent is 20% by weight or more, preferably 35% by weight or more, more preferably 40% by weight or more, further preferably 50% by weight or more, and particularly preferably 60% by weight or more, with respect to the total amount of the components of the preparation. The upper limit of the content of the volatile solvent is preferably 90% by weight or less, more preferably 85% by weight or less, further preferably 80% by weight or less, and particularly preferably 75% by weight or less, with respect to the total amount of the components of the preparation.

The volatile solvent may be used alone, or may be used in the form of a mixture of two or more types.

The volatile solvent preferably comprises at least one type of volatile solvent other than water.

The content of the volatile solvent other than water in the preparation of the present invention is preferably 50% by weight or more and 90% by weight or less, and more preferably 60% by weight or more and 80% by weight or less. The content of the volatile solvent other than water is further preferably 60% by weight or more and 75% by mass or less.

### (d) Surfactant

The surfactant described in the present description is not particularly limited, as long as it has a hydrophilic group and a hydrophobic group in a single molecule thereof, or it forms a micelle in a solvent. Examples of the surfactant include substances listed as surfactants in the website of Japan Surfactant Industry Association (http://www.jp-surfactant.jp/index.html), and substances classified into surfactants in Encyclopedia of Pharmaceutical Additives 2016 (edited by International Pharmaceutical Excipients Council Japan). The surfactants can be classified into a nonionic surfactant, an anionic surfactant, a cationic surfactant, and an amphoteric surfactant. Otherwise, the surfactants can also be classified into surfactants having a glycerin backbone or a propylene glycol backbone, and surfactants having a sorbitan backbone.

Examples of the nonionic surfactant include sorbitan monooleate, sorbitan monostearate, sorbitan tristearate, sorbitan monolaurate, sorbitan monopalmitate, sorbitan sesquioleate, sorbitan trioleate, glyceryl monooleate, glyceryl monostearate, glyceryl monomyristate, diglyceryl monooleate, decaglyceryl laurate, hexaglyceryl polyricinoleate, decaglyceryl pentastearate, α-monoisostearyl glyceryl ether, diglyceryl monoisostearate, diglyceryl monostearate, polyoxyethylene glycerin monostearate, polyoxyethylene glyceryl triisostearate, polyoxyethylene coconut oil fatty acid glyceryl, propylene glycol monostearate, ethylene glycol monostearate, polyoxyethylene (9) lauryl ether, polyoxyethylene (2) lauryl ether, polyoxyethylene (4,2) lauryl ether, polyoxyethylene (5) nonyl phenyl ether, polyoxyethylene (7,5) nonyl phenyl ether, polyoxyethylene (10) nonyl phenyl ether, polyoxyethylene (3) octyl phenyl ether, polyoxyethylene (10) octyl phenyl ether, polyoxyethylene oleyl ether, polyoxyethylene behenyl ether, polyoxyethylene stearyl ether, polyoxyethylene cetyl ether, polyoxyethylene cetostearyl ether, polyethylene glycol distearate, polyethylene glycol monostearate, polyoxyethylene lanoline, polyoxyethylene sorbitan monolaurate, polyoxyethylene sorbitan monostearate, polyoxyethylene sorbitan trioleate, polyoxyethylene sorbitan tetraoleate, polyoxyethylene sorbit beeswax, polyoxyethylene (10) oleylamine, polyoxy (5) oleylamine, polyoxy (5) oleamide, polyoxyethylene (2) monolaurate, polyoxyethylene castor oil (hydrogenated castor oil), polyoxyl stearate 40, Macrogol (registered trademark) 400, Cetomacrogol 1000, Lauromacrogol, polyoxyethylene (20) polyoxypropylene (20) glycol, polyoxyethylene (10) polyoxypropylene (4) cetyl ether, Polysorbate 20, Polysorbate 40, Polysorbate 60, Polysorbate 65, Polysorbate 80, polyethylene glycol monooleate, polyethylene glycol monolaurate, polyethylene glycol monostearate, diethanolamide laurate, hydrogenated soybean phospholipid, surfactin, dimethyl polysiloxane, cyclomethicone, methylphenyl polysiloxane, and lauryl pyrrolidone.

Examples of the anionic surfactant include sodium lauryl sulfate, potassium lauryl sulfate, triethanolamine lauryl sulfate, sodium cetyl sulfate, sodium lauroyl sarcosinate, sodium di-2-ethylhexyl sulfosuccinate, polyoxyethylene (10) lauryl ether sodium phosphate, polyoxyethylene sodium lauryl ether sulfate, dicetyl phosphate, sodium lauryl phosphate, polyoxyethylene (4) sodium lauryl ether phosphate, polyoxyethylene (5) cetyl ether sodium phosphate, polyoxyethylene (6) oleyl ether sodium phosphate, polyoxyethylene stearyl ether sodium phosphate, nonyl phenyl polyoxyethylene ether sulfate ammonium salt, polyoxyethylene oleyl ether diethanolamine phosphate, sodium N-cocoyl-N-methylaminoethylsulfonate (cocoyl methyl taurine sodium), sodium dodecylbenzenesulfonate, sodium N-acyl-L-glutamate, and surfactin sodium.

Examples of the cationic surfactant include stearyl trimethyl ammonium chloride, distearyl dimethyl ammonium chloride, benzalkonium chloride, stearyl dimethyl benzyl ammonium chloride, and N-cocoyl-L-arginine ethyl ether DL-pyrrolidone carboxylate (N-coconut oil fatty acid acyl-L-arginine-ethyl DL-pyrrolidone carboxylate).

Examples of the amphoteric surfactant include lauryldimethylaminoacetic acid betaine, 2-alkyl-N-carboxymethyl-N-hydroxyethyl imidazolinium betaine, disodium cocoamphodiacetate, lauric acid amidopropyl betaine, 2-[(3-dodecaneamidopropan-1-yl)dimethylammonio]acetate, and lauryl dimethylamine oxide.

Examples of the surfactant having a glycerin backbone include glyceryl monooleate, glyceryl monostearate, glyceryl monomyristate, diglyceryl monooleate, decaglyceryl laurate, hexaglyceryl polyricinoleate, decaglyceryl pentastearate, α-monoisostearyl glyceryl ether, diglyceryl monoisostearate, diglyceryl monostearate, polyoxyethylene glyceryl monostearate, polyoxyethylene glyceryl triisostearate, and polyoxyethylene coconut oil fatty acid glyceryl.

An example of the surfactant having a propylene glycol backbone is propylene glycol monostearate.

Examples of the surfactant having a sorbitan backbone include sorbitan monooleate, sorbitan monostearate, sorbitan tristearate, sorbitan monolaurate, sorbitan monopalmitate, sorbitan sesquioleate, sorbitan trioleate, polyoxyethylene sorbitan monolaurate, polyoxyethylene sorbitan monostearate, polyoxyethylene sorbitan trioleate, polyoxyethylene sorbitan tetraoleate, polyoxyethylene sorbit beeswax, Polysorbate 20, Polysorbate 40, Polysorbate 60, Polysorbate 65, and Polysorbate 80.

The above-classified surfactants may be used alone, or may also be used in the form of a mixture of two or more types.

The surfactant is preferably a cationic surfactant, and is particularly preferably N-coconut oil fatty acid acyl-L-arginine-ethyl DL-pyrrolidone carboxylate. A keratin protein which is a component constituting the nail is rich in negative charges (anions). Thus, it is assumed that a cationic surfactant interacts with the negative charges of keratin to destroy the conformation of the protein, thereby enhancing the permeability of a drug.

When the content of the surfactant is too high with respect to the total amount of the components of the preparation, the adhesiveness of the preparation to nails is reduced. The surfactant may be added to the preparation within a range that does not impair applicability or adhesiveness to nails. The content of the surfactant is 0.01% by weight or more and less than 30% by weight, more preferably 0.1% by weight or more and 25% by weight or less, further preferably 1% by weight or more and 20% by weight or less, particularly preferably 1% by weight or more and 15% by weight or less, and most preferably 1% by weight or more and 10% by weight or less, with respect to the total amount of the components of the preparation.

### (e) Other components

The preparation of the present invention may comprise non-volatile components such as (e1) esters, (e2) alcohols, (e3) a nitrogen-containing compound, (e4) organic acid, and (e5) terpenes, alone or in combination, as necessary. Besides, the term "non-volatile component" is used in the present description to mean a liquid or solid compound having a boiling point higher than a boiling point of water.

### (e1) Esters

Examples of the esters include: fatty acid monovalent alcohol esters, such as isopropyl isostearate, methyl stearate, butyl stearate, ethyl linoleate, isopropyl linoleate, ethyl oleate, oleyl oleate, decyl oleate, isopropyl myristate, butyl myristate, myristyl myristate, cetyl isooctanoate, octyldodecyl myristate, diethyl adipate, diisopropyl adipate, diisobutyl adipate, methyl laurate, hexyl laurate, methyl myristate, methyl caproate, methyl palmitate, cetyl palmitate, retinol palmitate, isopropyl palmitate, isostearyl palmitate, isopropyl myristate, diethyl sebacate, diisopropyl sebacate, cetyl 2-ethylhexanoate, hexadecyl isostearate, butyl acetate, or benzyl acetate; aromatic carboxylic acid monovalent alcohol esters, such as diethyl phthalate, dibutyl phthalate, or triethyl citrate; lactic acid esters, such as ethyl lactate, cetyl lactate, or myristyl lactate; carbonic acid esters, such as ethylene carbonate or propylene carbonate; and triacyl glycerol esters, such as triacetin, tricaprylin, a tri(caprylic-capric acid) glyceride-tristearate glyceride mixture, or tri(caprylic-capric acid)glycerin.

These esters may be used alone or in the form of a mixture of two or more types.

Such esters may be added to the preparation of the present invention within the range in which the preparation is easily applicable and does not cause stickiness on the surface. Among others, the amount of the esters is preferably 20% by weight or less, more preferably 15% by weight or less, further preferably 10% by weight or less, and particularly preferably 7% by weight or less, with respect to the total amount of the components of the preparation.

When the esters are used, the esters may be replaced with a part of the volatile solvent constituting the preparation, so that they may be added to the preparation.

### (e2) Alcohols

Examples of the alcohols include fatty acid alcohols, such as isostearyl alcohol, lauryl alcohol, 2-octyldodecanol, 2-hexyldecanol, cetanol, myristyl alcohol, oleyl alcohol, ethylene glycol, triethylene glycol, propylene glycol, dipropylene glycol, 1,3-butanediol, 1,2,6-hexanetriol, 2-ethyl-1,3-hexanediol, or glycerin; and aromatic alcohols, such as glycol salicylate or benzyl alcohol.

These alcohols may be used alone or in the form of a mixture of two or more types. Such alcohols may be added to the preparation within the range in which the preparation is easily applicable and does not cause stickiness on the surface. Among others, the amount of the alcohols is preferably 20% by weight or less, more preferably 15% by weight or less, further preferably 10% by weight or less, and particularly preferably 7% by weight or less, with respect to the total amount of the components of the preparation.

When the alcohols are used, the alcohols may be replaced with a part of the volatile solvent constituting the preparation, so that they may be added to the preparation.

### (e3) Nitrogen-containing compound

Examples of the nitrogen-containing compound include: aliphatic amines such as ethylenediamine, diisopropanolamine, diethanolamine, triisopropanolamine, or triethanolamine; amides such as N-methyl-2-pyrrolidone, crotamiton, or urea; and amino acids such as cysteine or methionine.

These nitrogen-containing compounds may be used alone or in the form of a mixture of two or more types. Such nitrogen-containing compounds may be added to the preparation within the range in which the preparation is easily applicable and does not cause stickiness on the surface. Among others, the amount of the nitrogen-containing compounds is preferably 20% by weight or less, more preferably 15% by weight or less, further preferably 10% by weight or less, and particularly preferably 7% by weight or less, with respect to the total amount of the components of the preparation.

When the nitrogen-containing compounds are used, the nitrogen-containing compounds may be replaced with a part of the volatile solvent constituting the preparation, so that they may be added to the preparation.

### (e4) Organic acid

Examples of the organic acid include: fatty acids, such as adipic acid, isostearic acid, caprylic acid, capric acid, acetic acid, stearic acid, lactic acid, palmitic acid, propionic acid, fumaric acid, behenic acid, maleic acid, or myristic acid; and aromatic carboxylic acids, such as benzoic acid, phthalic acid, or salicylic acid.

These organic acids may be used alone or in the form of a mixture of two or more types. Such organic acids may be added to the preparation within the range in which the preparation is easily applicable and does not cause stickiness on the surface. Among others, the amount of the organic acids is preferably 20% by weight or less, more preferably 15% by weight or less, further preferably 10% by weight or less, and particularly preferably 7% by weight or less, with respect to the total amount of the components of the preparation.

When the organic acids are used, the organic acids may be replaced with a part of the volatile solvent constituting the preparation, so that they may be added to the preparation.

### (e5) Terpenes

Examples of the terpenes include squalane, squalene, menthol, and borneol.

These terpenes may be used alone or in the form of a mixture of two or more types. Such terpenes may be added to the preparation within the range in which the preparation is easily applicable and does not cause stickiness on the surface. Among others, the amount of the terpenes is preferably 20% by weight or less, more preferably 15% by weight or less, further preferably 10% by weight or less, and particularly preferably 7% by weight or less, with respect to the total amount of the components of the preparation.

When the terpenes are used, the terpenes may be replaced with a part of the volatile solvent constituting the preparation, so that they may be added to the preparation.

The preparation according to the present invention may further comprise various types of known additives, such as a stabilizer, a perfume, a coloring agent, and a preservative, within the range that does not impair the effects of the present invention.

### [Vessel]

The vessel used in the present invention is not particularly limited, and a commonly used vessel can be used. Examples of the vessel used herein include an ointment jar made from polypropylene and an aluminum tube.

### [Method of preparing pharmaceutical composition used in production of preparation]

The preparation of the present invention can be prepared using the below-mentioned pharmaceutical composition. The pharmaceutical composition can be prepared by mixing and stirring the following components. That is to say, (a) efinaconazole or a salt thereof, (b) ethyl cellulose, (c) a volatile solvent, and (d) a surfactant are mixed and stirred to obtain a pharmaceutical composition. With regard to the order of mixing these components, for example, the component (a) to the component (d) may be successively added, or the component (b) and the component (c) may have previously been mixed with each other, and the component (a) and the component (d) may be then mixed with the obtained mixture. Individual components may be prepared in a vessel for storing a preparation, or the components may be prepared in each different vessels and may be then transferred into a storage vessel.

Upon the mixing and stirring of the components, the components may be heated, so as to facilitate the uniform mixing of the components. The heating temperature is preferably 30°C or higher, more preferably 40°C or higher, and most preferably 50°C or higher. In order to prevent deterioration or volatilization of the components of the preparation composition, the upper limit of the temperature is preferably 100°C or lower, and more preferably 90°C or lower.

In the above-described heating temperature range, the heating temperature is preferably 30°C or higher and 100°C or lower, and more preferably 40°C or higher and 90°C or lower.

### [Method for producing preparation]

As a method for preparing the preparation of the present invention, a method of filling a pharmaceutical composition in a storage vessel can be preferably applied.

### [Intended use of preparation]

The preparation of the present invention can be used as an agent for coating nails.

Since the coating agent of the present invention comprises efinaconazole or a salt thereof, it can be used as a therapeutic agent for onychomycosis.

The thickness of the present preparation applied onto the nail can be adjusted within a range that does not impair the effects of the therapeutic agent for onychomycosis or the ease of use as an agent for coating nails. The thickness of the preparation upon the application thereof may be 3 mm or less, or 2 mm or less, or 1 mm or less, or may also be 20 µm or more, or 50 µm or more, or 100 µm or more.

When the preparation of the present invention is applied onto the nail, it has appropriate adhesive strength. The appropriate adhesive strength means adhesive strength to such an extent that it is not peeled off, even if the surface of the nail, at least, after application of the preparation thereto, is turned to the ground.

When the preparation of the present invention is applied onto the nail, it has appropriate viscosity. The appropriate viscosity means viscosity to such an extent that the dropping of the preparation can hardly be confirmed by naked eyes, even if the surface of the nail, at least, immediately after application of the preparation thereto, is turned vertical to the ground.

When the thus applied preparation is removed, the preparation may be picked with fingers or tweezers and may be then peeled. When the preparation is removed after the application thereof, from the viewpoint of drying even the inner portion of the applied preparation that is not contacted with the air, and also, from the viewpoint of sufficiently delivering the active ingredient into the nail, the removing timing is preferably 1 hour or more after the application of the preparation, more preferably two hours or more after the application thereof, further preferably 6 hours or more after the application thereof, particularly preferably 12 hours or more after the application thereof, and most preferably 24 hours or more after the application thereof.

The preparation of the present invention does not have stickiness on the surface of the preparation after solidification thereof. On the other hand, the present preparation has sufficient adhesive strength to the applied surface, and thus, the preparation is not easily dropped from the applied surface, before the peeling operation is carried out.

### Examples

Hereinafter, the present invention will be more specifically described in Examples, Test Examples, and Preparation Examples. However, these examples are not intended to limit the scope of the present invention. It is to be noted that the numerical value of each component shown in Tables 1 to 5 and Table 7 indicates part by mass. The numerical value of each component shown in Table 8 indicates % by mass.

### (Evaluation Method) Film formation time

From the following viewpoints, the time required for the formation of a film from the preparation was evaluated.
○: After application of the preparation, the time required until solidification of the surface was less than 10 minutes.
×: After application of the preparation, the time required until solidification of the surface was 10 minutes or more.

### (Evaluation Method) peeling ability

From the following viewpoints, the peeling ability of the preparation was evaluated.
○: When the preparation was peeled off, it was peeled off as an aggregate, without particular disintegration.
×: When the preparation was peeled off, the preparation remained on the applied surface and was disintegrated.

### (Evaluation Method) Stickiness on the surface

From the following viewpoints, the stickiness of the preparation was evaluated.
○: Sixty minutes after application of the preparation, stickiness was not felt, even touching the surface of the preparation.
Δ: Sixty minutes after application of the preparation, slight stickiness was felt, when touching the surface of the preparation.
×: Sixty minutes after application of the preparation, stickiness was felt, when touching the surface of the preparation.

### (Evaluation Method) Evaluation of permeability through human nails

A dead human nail was immersed in an infiltrate (e.g., a 5% ethanol-containing 0.01 mol/L phosphate buffered saline (pH 7.2 to 7.4)) for 30 minutes or more, and thereafter, the nail was cut into a section with a diameter of approximately 5 to 6 mm. The nail plate side of the cut nail was attached along a rubber-made O-ring with an inner diameter of 4 mm, and the gap was then caulked with an adhesive. The upper and lower sides thereof were sandwiches between the processed plastic-made screw vial and screw cap, and were then equipped into an automatic sampling apparatus (manufactured by CosMED Pharmaceutical Co. Ltd.; TransView C12). As a receptor solution, a 5% ethanol-containing 0.01 mol/L phosphate buffered saline (pH 7.2 to 7.4) was used, and the receptor temperature was set at 32°C. Twenty-four hours after initiation of the test, an aliquot of the receptor solution was sampled, and the amount of efinaconazole permeating through the human nail in the receptor solution was then quantified by LC/MS/MS. As a control preparation, Clenafin (registered trademark) (manufactured by Kaken Pharmaceutical Co., Ltd.; 10% Efinaconazole Solution Preparation) was used.

### (Example 1)

0.32 g of Ethyl cellulose (ethyl group substitution percentage: 48.0% to 49.5%; weight average molecular weight: 180000), 1.28 g of ethanol, 0.20 g of efinaconazole, and 0.20 g of AMPHITOL (registered trademark) 20AB (20% - 30% lauric acid amidopropyl betaine solution) were successively added into a polypropylene-made ointment jar with a volume of 6 mL, and they were then mixed and stirred using a stirring degassing machine at 2000 rpm for 15 minutes. Thereafter, the resulting mixture was degassed at 2200 rpm for 90 seconds, to obtain a homogeneous pharmaceutical composition. The components of the preparation and the weight percentage (%) thereof are shown in Table 1.

### (Example 2)

A preparation was produced by the same method as that of Example 1, with the exception that efinaconazole, ethyl cellulose, ethanol, and surfactin sodium were mixed with one another at the ratio shown in Table 1.

### (Comparative Examples 1 to 4)

In the case of a film-forming preparation, in order to ensure the cohesiveness (peeling ability) of the preparation after completion of the film formation, a predetermined amount of non-volatile plasticizer is added in many cases. As common plasticizers, esters and alcohols have been known.

A preparation was produced by the same method as that of Example 1, with the exception that efinaconazole, ethyl cellulose, ethanol, and ester or alcohol were mixed with one another at the ratio shown in Table 1.

### (Ethyl cellulose)

Ethyl cellulose A: ethyl group substitution percentage: 48.0% - 49.5%; weight average molecular weight: 180000

Besides, since the ethyl group substitution percentage and the weight average molecular weight may be changed depending on the production lot of ethyl cellulose used, the values described in the present description are merely representative values, and thus, are not necessarily limited thereto.

**[Table 1]**

| | | Ex. 1 | Ex. 2 | Comp. Ex. 1 | Comp. Ex. 2 | Comp. Ex. 3 | Comp. Ex.4 |
|---|---|---|---|---|---|---|---|
| Drug substance | Efinaconazole | 10 | 10 | 10 | 10 | 10 | 10 |
| Base | Ethyl cellulose A | 16 | 16 | 16 | 16 | 16 | 16 |
| Volatile solvent | Ethanol | 64 | 69 | 74 | 64 | 64 | 64 |
| Surfactant | AMPHITOL 20AB | 10 | | | | | |
| | Surfactin sodium | | 5 | | | | |
| Alcohol | 2-Hexyl-1-decanol | | | | 10 | | |
| Ester | Triacetin | | | | | 10 | |
| | Diisopropyl adipate | | | | | | 10 |
| Film formation time | | ○ | ○ | ○ | ○ | ○ | ○ |
| Peeling ability | | ○ | ○ | × | × | ○ | × |
| Surface stickiness | | ○ | ○ | ○ | × | × | × |

Stickiness on the surface was not observed in both of the preparations of Examples 1 and 2, to which a surfactant had been added. In addition, in Examples 1 and 2, the preparation could be removed without performing a special operation of washing the preparation with water, a solvent or the like, and also, the preparation did not remain after the peeling. In Comparative Example 1, in which a non-volatile component other than the drug and the base had not been added to the preparation, the preparation was disintegrated upon the peeling thereof. In Comparative Examples 2 to 4, in which alcohols or esters had been added, instead of a surfactant, stickiness was observed on the surface of the preparation.

### (Examples 3 to 12)

A preparation was produced by the same method as that of Example 1, with the exception that efinaconazole, ethyl cellulose, ethanol, and a surfactant were mixed with one another at the ratio shown in Table 2.

**[Table 2]**

| | | Ex. 3 | Ex. 4 | Ex. 5 | Ex. 6 | Ex. 7 | Ex. 8 |
|---|---|---|---|---|---|---|---|
| Drug substance | Efinaconazole | 10 | 10 | 10 | 10 | 10 | 10 |
| Base | Ethyl cellulose A | 16 | 16 | 16 | 16 | 16 | 16 |
| Volatile solvent | Ethanol | 64 | 64 | 64 | 64 | 64 | 64 |
| Surfactant | Polyoxyethylene sorbitan monooleate | 10 | | | | | |
| | Diglyceryl monooleate | | 10 | | | | |
| | coconut oil fatty acid diethanol amide solution | | | 10 | | | |
| | Lauromacrogol | | | | 10 | | |
| | Polyoxyethylene coconut oil fatty | | | | | 10 | |
| | acid sorbitan | | | | | | |
| | Polyethylene glycol monooleate | | | | | | 10 |
| Film formation time | | ○ | ○ | ○ | ○ | ○ | ○ |
| Peeling ability | | ○ | ○ | ○ | ○ | ○ | ○ |
| Surface stickiness | | ○ | ○ | ○ | ○ | ○ | ○ |
| | | | | | | | |

| | | Ex. 9 | Ex. 10 | Ex. 11 | Ex. 12 | | |
|---|---|---|---|---|---|---|---|
| Drug substance | Efinaconazole | 10 | 10 | 10 | 10 | | |
| Base | Ethyl cellulose A | 16 | 16 | 16 | 16 | | |
| Volatile solvent | Ethanol | 71 | 64 | 64 | 64 | | |
| Surfactant | N-coconut oil fatty acid acyl-L-arginine ethyl DL-pyrrolidone carboxylate | 3 | | | | | |
| | 30.5% Sodium lauroyl sarcosinate | | 10 | | | | |
| | 25% Sodium methyl cocoyl taurate | | | 10 | | | |
| | Polyoxyethylene lauryl ether sodium phosphate | | | | 10 | | |
| Film formation time | | ○ | ○ | ○ | ○ | | |
| Peeling ability | | ○ | ○ | ○ | ○ | | |
| Surface stickiness | | ○ | ○ | ○ | ○ | | |

With regard to all of the preparations in Examples 3 to 8, in which a nonionic surfactant had been added, in Example 9, in which a cationic surfactant had been added, and in Examples 10 to 12, in which an anionic surfactant had been added, no stickiness was observed on the surface of the preparation. Moreover, in Examples 3 to 12, the preparation could be removed without performing a special operation of washing the preparation with water, a solvent or the like, and also, the preparation did not remain after the peeling.

### (Examples 13 to 17 and Comparative Example 5)

A preparation was produced by the same method as that of Example 1, with the exception that efinaconazole, ethyl cellulose, ethanol, and a surfactant were mixed with one another at the ratio shown in Table 3.

**[Table 3]**

| | | Ex. 13 | Ex. 14 | Ex. 15 | Ex. 16 | Ex. 17 | Comp. Ex. 5 |
|---|---|---|---|---|---|---|---|
| Drug substance | Efinaconazole | 10 | 10 | 10 | 10 | 10 | 10 |
| Base | Ethyl cellulose A | 16 | 16 | 16 | 16 | 16 | 16 |
| Volatile solvent | Ethanol | 73 | 73 | 71 | 54 | 54 | 44 |
| Surfactant | Surfactin sodium | | | 3 | | | |
| | Polyoxyethylene coconut oil fatty acid sorbitan | 1 | | | 20 | | 30 |
| | Polyoxyethylene lauryl ether sodium phosphate | | 1 | | | 20 | |
| Film formation time | | ○ | ○ | ○ | ○ | ○ | - |
| Peeling ability | | ○ | ○ | ○ | ○ | ○ | - |
| Surface stickiness | | ○ | ○ | ○ | ○ | ○ | - |

In all cases where the additive amounts of the surfactant were set at 1% by weight, 3% by weight and 20% by weight, with respect to the total amount of the components of the preparation (Example 13 to 17), stickiness was not observed on the surface of the preparation. Moreover, in Examples 13 to 17, the preparation could be removed without performing a special operation of washing the preparation with water, a solvent or the like, and also, the preparation did not remain after the peeling. When the surfactant was used in an additive amount of 30% by weight with respect to the total amount of the components of the preparation, ethyl cellulose was hardly dissolved upon the production thereof, and the adhesiveness of the preparation to the applied surface was decreased, and it became unusable as a preparation.

### (Examples 18 and 19)

A preparation comprising a surfactant and an ester serving as a non-volatile component was produced. A preparation was produced by the same method as that of Example 1, with the exception that efinaconazole, ethyl cellulose, ethanol, a surfactant, and an ester were mixed with one another at the ratio shown in Table 4.

**[Table 4]**

| | | Ex. 18 | Ex.19 |
|---|---|---|---|
| Drug substance | Efinaconazole | 10 | 10 |
| Base | Ethyl cellulose A | 16 | 16 |
| Volatile solvent | Ethanol | 64 | 64 |
| Surfactant | Polyoxyethylene lauryl ether sodium phosphate | 5 | 3 |
| Ester | Triacetin | 5 | 7 |
| Film formation time | | ○ | ○ |
| Peeling ability | | ○ | ○ |
| Surface stickiness | | ○ | ○ |

In the case of the preparation containing a surfactant, even if an ester was comprised in the preparation in an amount of 5% by weight or 7% by weight with respect to the total amount of the components in the preparation (Example 18 and Example 19), a preparation having no stickiness on the surface could be obtained. In addition, in Examples 18 and 19, the preparation could be removed without performing a special operation of washing the preparation with water, a solvent or the like, and also, the preparation did not remain after the peeling (Comparative Examples 6 and 7).

As a generally known base for film-forming preparations, hydroxypropyl cellulose was selected instead of ethyl cellulose. A preparation was produced by the same method of that of Example 1, with the exception that efinaconazole, hydroxypropyl cellulose, ethanol, and a surfactant were mixed with one another at the ratio shown in Table 5.

**[Table 5]**

| | | Comp. Ex. 6 | Comp. Ex.7 |
|---|---|---|---|
| Drug substance | Efinaconazole | 10 | 10 |
| Base | Hydroxypropyl cellulose | 16 | 16 |
| Volatile solvent | Ethanol | 74 | 64 |
| Surfactant | AMPHITOL 20AB | | 10 |
| Film formation time | | × | × |
| Peeling ability | | × | ○ |
| Surface stickiness | | × | × |

When hydroxypropyl cellulose was used as a base, stickiness was observed on the surface of the preparation.

### (Example 20)

The results obtained by measuring the amount of the drug permeating through nails are shown in Table 6.

**[Table 6]**

| | |
|---|---|
| Clenafin | 0.4 µg/cm² |
| Example 1 | 0.8 µg/cm² |

The amount of efinaconazole permeating through human nails in the preparation of Example 1 was approximately 2 times larger than the amount of Clenafin which is an existing preparation.

### (Example 21)

A preparation was produced by the same method as that of Example 1, with the exception that efinaconazole, ethyl cellulose, ethanol, triacetin, and a surfactant were mixed with one another at the ratio shown in Table 7.

### (Examples 22 and 23)

A preparation was produced by the same method as that of Example 1, with the exception that efinaconazole, ethyl cellulose, ethanol, and a surfactant were mixed with one another at the ratio shown in Table 7.

**[Table 7]**

| | | Example 21 | Example 22 | Example 23 |
|---|---|---|---|---|
| Drug substance | Efinaconazole | 10 | 10 | 10 |
| Base | Ethyl cellulose A | 16 | 16 | 16 |
| Volatile solvent | ethanol | 61 | 73 | 71 |
| Surfactant | Polyoxyethylene lauryl ether sodium phosphate | 3 | 1 | 3 |
| Ester | Triacetin | 10 | | |
| Film formation time | | ○ | ○ | ○ |
| Peeling ability | | ○ | ○ | ○ |
| Surface stickiness | | Δ | ○ | ○ |

In Example 21, in which an ester was comprised in the preparation in amount of 10% by mass with respect to the total amount of the components in the preparation, slight stickiness was observed on the surface, but such stickiness was at a level causing no practical problems.

In Examples 22 and 23, in which a surfactant was comprised in the preparation in amount of 1% by mass or 3% by mass with respect to the total amount of the components in the preparation, both of the preparations were found to have no stickiness on the surface, and the preparations could be removed without performing a special operation of washing the preparation with water, a solvent or the like, and also, the preparations did not remain after the peeling.

### (Example 24)

The results obtained by measuring the amount of the drug permeating through nails are shown in Table 8.

**[Table 8]**

| | |
|---|---|
| Clenafin | 0.135 µg/cm² |
| Example 9 | 0.535 µg/cm² |

## Claims

1. A therapeutic film-forming preparation for onychomycosis, comprising:
a) efinaconazole or a salt thereof,
b) 1% by weight or more and 30% by weight or less of ethyl cellulose,
c) 20% by weight or more of a volatile solvent, wherein the volatile solvent is water or a liquid component having a boiling point lower than a boiling point of water, and
d) 0.01% by weight or more and less than 30% by weight of a surfactant, preferably 0.1% by weight or more and 25% by weight or less of a surfactant, more preferably 1% by weight or more and 20% by weight or less of a surfactant.

2. The preparation according to claim 1, wherein an amount of the ethyl cellulose in the preparation is 5% by weight or more and 25% by weight or less, preferably 10% by weight or more and 20% by weight or less.

3. The preparation according to claim 1 or 2, wherein an amount of the efinaconazole or a salt thereof in the preparation is 0.1% by weight or more and 30% by weight or less, preferably 0.5% by weight or more and 25% by weight or less, more preferably 1% by weight or more and 20% by weight or less.

4. The preparation according to any one of claims 1 to 3, wherein the surfactant comprises a cationic surfactant, preferably wherein the cationic surfactant is N-coconut oil fatty acid acyl-L-arginine-ethyl DL-pyrrolidone carboxylate.

5. The preparation according to any one of claims 1 to 4, wherein the surfactant comprises an anionic surfactant.

6. The preparation according to any one of claims 1 to 5, wherein the surfactant comprises an amphoteric surfactant.

7. The preparation according to any one of claims 1 to 6, wherein the surfactant comprises a nonionic surfactant.

8. The preparation according to any one of claims 1 to 7, wherein the surfactant has a glycerin backbone or a propylene glycol backbone or a sorbitan backbone.

9. The preparation according to any one of claims 1 to 8, wherein the volatile solvent comprises at least one selected from the group consisting of ethanol, isopropanol, ethyl acetate, acetone, diethyl ether, dimethyl ether, and water.

10. The preparation according to any one of claims 1 to 9, wherein the volatile solvent comprises at least one volatile solvent other than water, preferably wherein an amount of the at least one volatile solvent other than water in the preparation is 50% by weight or more and 90% by weight or less.

11. The preparation according to any one of claims 1 to 10, further comprising a non-volatile component other than the surfactant, preferably wherein an amount of the non-volatile component other than the surfactant in the preparation is 7% by weight or less.

12. The preparation according to claim 11, wherein the non-volatile component other than the surfactant comprises an ester.

13. The preparation according to claim 11, wherein the non-volatile component other than the surfactant comprises an alcohol.

14. The preparation according to any one of claims 1 to 13, which is a liquid agent for external use, and is a film forming agent that solidifies after it has been applied to an effected area.

## Patentansprüche

1. Therapeutische filmbildende Zubereitung für Onychomykose, umfassend:
a) Efinaconazol oder ein Salz davon,
b) 1 Gew.-% oder mehr und 30 Gew.-% oder weniger Ethylcellulose,
c) 20 Gew.-% oder mehr eines flüchtigen Lösungsmittels, wobei das flüchtige Lösungsmittel Wasser oder eine flüssige Komponente ist, die einen Siedepunkt aufweist, der niedriger als ein Siedepunkt von Wasser ist, und
d) 0,01 Gew.-% oder mehr und weniger als 30 Gew.-% eines Tensids, vorzugsweise 0,1 Gew.-% oder mehr und 25 Gew.-% oder weniger eines Tensids, besonders bevorzugt 1 Gew.-% oder mehr und 20 Gew.-% oder weniger eines Tensids.

2. Zubereitung gemäß Anspruch 1, wobei eine Menge der Ethylcellulose in der Zubereitung 5 Gew.-% oder mehr und 25 Gew.-% oder weniger, vorzugsweise 10 Gew.-% oder mehr und 20 Gew.-% oder weniger, beträgt.

3. Zubereitung gemäß Anspruch 1 oder 2, wobei eine Menge des Efinaconazols oder eines Salzes davon in der Zubereitung 0,1 Gew.-% oder mehr und 30 Gew.-% oder weniger, vorzugsweise 0,5 Gew.-% oder mehr und 25 Gew.-% oder weniger, besonders bevorzugt 1 Gew.-% oder mehr und 20 Gew.-% oder weniger, beträgt.

4. Zubereitung gemäß irgendeinem der Ansprüche 1 bis 3, wobei das Tensid ein kationisches Tensid umfasst, wobei das kationische Tensid vorzugsweise N-Kokosnussölfettsäure-Acyl-L-Arginin-Ethyl-DL-Pyrrolidoncarboxylat ist.

5. Zubereitung gemäß irgendeinem der Ansprüche 1 bis 4, wobei das Tensid ein anionisches Tensid umfasst.

6. Zubereitung gemäß irgendeinem der Ansprüche 1 bis 5, wobei das Tensid ein amphoteres Tensid umfasst.

7. Zubereitung gemäß irgendeinem der Ansprüche 1 bis 6, wobei das Tensid ein nicht-ionisches Tensid umfasst.

8. Zubereitung gemäß irgendeinem der Ansprüche 1 bis 7, wobei das Tensid ein Glyceringrundgerüst oder ein Propylenglykolgrundgerüst oder ein Sorbitangrundgerüst aufweist.

9. Zubereitung gemäß irgendeinem der Ansprüche 1 bis 8, wobei das flüchtige Lösungsmittel mindestens eines, ausgewählt aus der Gruppe, bestehend aus Ethanol, Isopropanol, Ethylacetat, Aceton, Diethylether, Dimethylether und Wasser, umfasst.

10. Zubereitung gemäß irgendeinem der Ansprüche 1 bis 9, wobei das flüchtige Lösungsmittel mindestens ein von Wasser verschiedenes flüchtiges Lösungsmittel umfasst, wobei eine Menge des mindestens einen von Wasser verschiedenen flüchtigen Lösungsmittels in der Zubereitung vorzugsweise 50 Gew.-% oder mehr und 90 Gew.-% oder weniger beträgt.

11. Zubereitung gemäß irgendeinem der Ansprüche 1 bis 10, ferner umfassend eine von dem Tensid verschiedene nicht-flüchtige Komponente, wobei eine Menge der von dem Tensid verschiedenen nicht-flüchtigen Komponente in der Zubereitung vorzugsweise 7 Gew.-% oder weniger beträgt.

12. Zubereitung gemäß Anspruch 11, wobei die von dem Tensid verschiedene nicht-flüchtige Komponente einen Ester umfasst.

13. Zubereitung gemäß Anspruch 11, wobei die von dem Tensid verschiedene nicht-flüchtige Komponente einen Alkohol umfasst.

14. Zubereitung gemäß irgendeinem der Ansprüche 1 bis 13, die ein flüssiges Mittel zur äußerlichen Anwendung ist und ein filmbildendes Mittel ist, das sich verfestigt, nachdem es auf einen betroffenen Bereich aufgetragen wurde.

## Revendications

1. Préparation filmogène thérapeutique pour l'onychomycose, comprenant :
a) de l'éfinaconazole ou un sel de celui-ci,
b) 1 % en poids ou plus et 30 % en poids ou moins d'éthyle cellulose,
c) 20 % en poids ou plus d'un solvant volatil, dans laquelle le solvant volatil est l'eau ou un composant liquide présentant un point d'ébullition plus bas qu'un point d'ébullition de l'eau, et
d) 0,01% en poids ou plus et moins de 30 % en poids d'un tensioactif, de préférence 0,1% en poids ou plus et 25% en poids ou moins d'un tensioactif, plus préférablement 1 % en poids ou plus et 20 % en poids ou moins d'un tensioactif.

2. Préparation selon la revendication 1, dans laquelle une quantité d'éthylcellulose dans la préparation est de 5 % en poids ou plus et 25 % en poids ou moins, de préférence de 10 % en poids ou plus et 20 % en poids ou moins.

3. Préparation selon la revendication 1 ou 2, dans laquelle une quantité de l'éfinaconazole ou d'un sel de celui-ci dans la préparation est de 0,1 % en poids ou plus et 30 % en poids ou moins, de préférence 0,5 % en poids ou plus et 25 % en poids ou moins, plus préférablement 1 % en poids ou plus et 20 % en poids ou moins.

4. Préparation selon l'une quelconque des revendications 1 à 3, dans laquelle le tensioactif comprend un tensioactif cationique, de préférence dans laquelle le tensioactif cationique est le carboxylate d'éthyl-L-arginine de N-acyle d'acide gras d'huile de noix de coco DL-pyrrolidone.

5. Préparation selon l'une quelconque des revendications 1 à 4, dans laquelle le tensioactif comprend un tensioactif anionique.

6. Préparation selon l'une quelconque des revendications 1 à 5, dans laquelle le tensioactif comprend un tensioactif amphotère.

7. Préparation selon l'une quelconque des revendications 1 à 6, dans laquelle le tensioactif comprend un tensioactif non ionique.

8. Préparation selon l'une quelconque des revendications 1 à 7, dans laquelle le tensioactif présente un squelette glycérol ou un squelette propylène glycol ou un squelette sorbitane.

9. Préparation selon l'une quelconque des revendications 1 à 8, dans laquelle le solvant volatil comprend au moins l'un sélectionné parmi le groupe consistant en de l'éthanol, de l'isopropanol, de l'acétate d'éthyle, de l'acétone, de l'éther diéthylique, de l'éther diméthylique et de l'eau.

10. Préparation selon l'une quelconque des revendications 1 à 9, dans laquelle le solvant volatil comprend au moins un solvant volatil autre que l'eau, de préférence dans laquelle une quantité de l'au moins un solvant volatil autre que l'eau dans la préparation est de 50 % en poids ou plus et 90 % en poids ou moins.

11. Préparation selon l'une quelconque des revendications 1 à 10, comprenant en outre un composant non volatil autre que le tensioactif, de préférence dans laquelle une quantité du composant non volatil autre que le tensioactif dans la préparation est de 7 % en poids ou moins.

12. Préparation selon la revendication 11, dans laquelle le composant non volatil autre que le tensioactif comprend un ester.

13. Préparation selon la revendication 11, dans laquelle le composant non volatil autre que le tensioactif comprend un alcool.

14. Préparation selon l'une quelconque des revendications 1 à 13, qui est un agent liquide pour utilisation externe, et est un agent filmogène qui se solidifie après avoir été appliqué sur une zone concernée.
